# EUROPÄISCHE PATENTSCHRIFT

(11) **EP 3 542 703 B1**
(45) Veröffentlichungstag und Bekanntmachung des Hinweises auf die Patenterteilung: **07.04.2021**
(21) Anmeldenummer: 19156430.1
(22) Anmeldetag: 11.02.2019
(51) Int. Cl.: A61B 3/024, A61B 3/113, A61H 5/00

(54) **VORRICHTUNG UND VERFAHREN ZUM ERFASSEN EINES GESICHTSFELDS EINER EIN SKOTOM AUFWEISENDEN PERSON**
APPARATUS AND METHOD FOR DETECTING A FIELD OF VIEW OF A PERSON WITH A BLIND SPOT
DISPOSITIF ET PROCÉDÉ DE DÉTECTION D'UN CHAMP VISUEL D'UNE PERSONNE COMPORTANT UN SCOTOME

(30) Priorität: 16.03.2018 DE 102018106125
(43) Veröffentlichungstag der Anmeldung: 25.09.2019
(73) Patentinhaber: Carl Zeiss Vision International GmbH, 73430 Aalen (DE)
(72) Erfinder: WAHL, Siegfried, 73072 Donzdorf (DE); RIFAI, Katharina, 72072 Tübingen (DE); BARRAZA-BERNAL, Maria Jose, 73431 Aalen (DE)
(74) Vertreter: Walcher, Armin

(56) Entgegenhaltungen:
- WO-A1-2016/182514
- WO-A1-2017/001335
- JP-A- 2006 280 665
- US-A1- 2016 198 941

## Beschreibung

Die Erfindung betrifft eine Vorrichtung und ein Verfahren zum Erfassen eines Gesichtsfelds einer ein Skotom aufweisenden Person. Bei einem Skotom handelt es sich um einen Teilbereich des Gesichtsfeldes, dessen Sensibilität - meist krankhaft - herabgesetzt oder vollständig verloren ist. Skotome können durch eine Erkrankung der Netzhaut, der Sehbahn und/oder des Sehzentrums auftreten.

Die Erfindung betrifft insbesondere ein Zentralskotom. Ein Zentralskotom bezieht sich auf einen vollständigen oder teilweisen Verlust des zentralen Gesichtsfelds, also auf einen Sensibilitätsverlust im Bereich der Makula, insbesondere im Bereich der Fovea.

Der Verlust des zentralen Gesichtsfelds wird normalerweise durch die Verwendung verbleibender gesunder Bereiche der Netzhaut kompensiert. Ein solcher Bereich wird als bevorzugter retinaler Fixationsort, *engl. preferred retinal locus (PRL) for fixation,* bezeichnet.

Es ist bekannt, dass Patienten bevorzugte retinale Fixationsorte nicht immer in Bereichen ausbilden, die für eine spezifische Sehaufgabe optimal sind. Beispielsweise bilden Patienten häufig einen bevorzugten retinalen Fixationsort auf der linken Seite des Skotoms aus. Dieser Bereich ist jedoch für das Lesen eines Textes von links nach rechts nicht vorteilhaft.

Weiterhin spielt auch die Form des Skotoms eine wichtige Rolle für die Bewältigung spezifischer Sehaufgaben. Die Form von Skotomen unterscheidet sich zwischen verschiedenen Patienten und kann sich außerdem über die Zeit hinweg ändern. Daher lässt sich kein allgemein gültiger optimaler bevorzugter retinaler Fixationsort bestimmen, der für jeden Patienten anwendbar wäre. Der optimale bevorzugte retinale Fixationsort ist vielmehr spezifisch für jeden Patienten auf der Grundlage der Form des Skotoms und der spezifischen Sehaufgabe zu bestimmen.

Aus der Veröffentlichung EP 3 111 828 A1 ist bekannt, dass die Lage des bevorzugten retinalen Fixationsorts durch verschiedene Trainingsstrategien beeinflusst werden kann. Dadurch kann der bevorzugte retinale Fixationsort von einem ineffizienten Ort zu einem effizienten Ort verlagert werden.

Zum Vermessen des Gesichtsfelds eines Patienten sind verschiedene Verfahren bekannt, beispielsweise die Perimetrie (Goldmann Perimetrie, Mikroperimetrie) oder Kampimetrie. Diese Verfahren liefern jedoch nicht ausreichend Information über die Form von Skotomen. Daher ist mit diesen Verfahren eine Bestimmung eines optimalen bevorzugten retinalen Fixationsorts nicht in Abhängigkeit der Form des Skotoms möglich.

Weiterhin werden bildgebende Verfahren, wie beispielsweise die Scanning-Laser-Ophthalmoskopie (SLO), die optische Kohärenztomografie (*engl. optical coherence tomography, OCT*) oder die Fundusfotografie, zur Abbildung der Netzhaut verwendet. Mit diesen Verfahren ist es möglich, beschädigte Bereiche der Netzhaut und deren Form in einer objektiven Weise zu bestimmen. Es ist jedoch nicht möglich, das vom Patienten subjektiv wahrgenommene Gesichtsfeld zu bestimmen.

Die internationale Patentanmeldung WO 2017/001 335 A1 betrifft eine Vorrichtung und ein Computerprogramm zum Trainieren eines bevorzugten retinalen Fixationsorts.

Die japanische Patentanmeldung JP 2006 280 665 A betrifft einen Perimeter.

Die US 2016/198941 A1 betrifft ein Verfahren zum Erfassen eines Gesichtsfelds einer ein Skotom aufweisenden Person.

Der vorliegenden Erfindung liegt die Aufgabe zugrunde, die Nachteile aus dem Stand der Technik zu beseitigen. Insbesondere sollen eine Vorrichtung und ein Verfahren bereitgestellt werden, womit Skotome im Gesichtsfeld eines Patienten besonders präzise erfasst werden und der Patient besonders präzise über das Skotom informiert wird. Damit soll die Wirksamkeit des Trainings des bevorzugten retinalen Fixationsorts verbessert werden.

Erfindungsgemäß wird diese Aufgabe verfahrensmäßig mit dem Gegenstand des Anspruchs 1 und vorrichtungsmäßig mit dem Gegenstand des Anspruchs 13 gelöst. Vorteilhafte Ausgestaltungen der Erfindung sind in den abhängigen Ansprüchen angegeben.

Das erfindungsgemäße Verfahren zum Erfassen eines Gesichtsfelds einer ein Skotom, insbesondere ein Zentralskotom, aufweisenden Person, umfasst die folgenden Schritte:
Fortwährendes Erfassen der Augenausrichtung der Person mittels einer Erfassungseinheit, punktweises Abtasten des Gesichtsfelds der Person, um sehtaugliche und sehuntaugliche Punkte im Gesichtsfeld der Person zu bestimmen,
Auffinden des Skotoms als einen Bereich mit einer Vielzahl von sehuntauglichen Punkten,
Berechnen einer äußeren Grenzlinie des Skotoms,
Berechnen einer äußeren Hüllkurve, die die äußere Grenzlinie des Skotoms in einem vorbestimmten Abstand umgibt,
Anzeigen der äußeren Hüllkurve auf einer Anzeigeeinheit unter Berücksichtigung der fortwährend erfassten Augenausrichtung derart, dass die äußere Hüllkurve für die Person bei beliebiger Ausrichtung des Auges der Person auf die Anzeigeeinheit als Umrahmung des Skotoms wahrnehmbar ist.

Erfindungsgemäß kann die Person also die äußere Hüllkurve mit Bereichen der intakten Netzhaut wahrnehmen, die das Skotom unmittelbar umgeben. Die äußere Hüllkurve umrahmt also in der Wahrnehmung der Person den Bereich des Skotoms. Nach einer vorteilhaften Ausgestaltung der Erfindung wird die äußere Hüllkurve auf der Anzeigeeinheit derart unter Berücksichtigung der fortwährend erfassten Augenausrichtung angezeigt, dass die äußere Hüllkurve für die Person über zuvor verfahrensgemäß bestimmten sehtauglichen Punkte im Gesichtsfeld, die in unmittelbarer Nachbarschaft der das Skotom bildenden sehuntauglichen Punkte liegen, wahrnehmbar ist.

Durch das erfindungsgemäße Verfahren können insbesondere die Lage, Größe und/oder Form des Skotoms der Person bestimmt werden.

Das erfindungsgemäße Verfahren ist sowohl bei monokularem Sehen (mit einem Auge) als auch bei binokularem Sehen (mit beiden Augen zugleich) anwendbar. Für eine Anwendung bei monokularem Sehen kann das zweite Auge beispielsweise abgedeckt sein. Die nachfolgende Beschreibung bezieht sich in ihrer Wortwahl auf den Fall des monokularen Sehens. Es versteht sich jedoch von selbst, dass die Begriffe "ein Auge" bzw. "das Auge" auch mit der Bedeutung "beide Augen" bzw. "beide Augen zugleich" gelesen werden kann.

Bei dem erfindungsgemäßen Verfahren kann das Gesichtsfeld der Person vollständig oder auch nur teilweise erfasst werden. Vorzugsweise wird das Gesichtsfeld im Bereich des Skotoms und in das Skotom unmittelbar umgebenden Bereichen (also in unmittelbarer Nachbarschaft des Skotoms) erfasst.

Das punktweise Abtasten des Gesichtsfelds der Person erfolgt vorzugsweise mittels der Anzeigeeinheit. Die Anzeigeeinheit ist vorzugsweise in einer definierten räumlichen Beziehung zur Position des Auges der Person angeordnet.

Der Begriff "punktweises Abtasten" ist im Sinne der Erfindung nicht auf den eigentlichen Wortsinn eingeschränkt zu verstehen. Vorzugsweise wird das Gesichtsfeld für das punktweise Abtasten in einzelne Sektoren eines Polarkoordinatensystems aufgeteilt. Vorzugsweise werden die einzelnen Sektoren nacheinander abgetastet. Daher kann der Begriff "punktweises Abtasten" auch durch den Begriff "sektorweises Abtasten" oder durch den Begriff "sequentielles Abtasten" ersetzt werden. Weiterhin kann der Begriff "Abtasten" durch die Begriffe "Detektieren" oder "Messen" bzw. "Vermessen" ersetzt werden. Im Sinne der Erfindung bezieht sich der Begriff "Abtasten" nicht auf ein kontinuierliches Überstreichen einer Linie bzw. einer Fläche, sondern auf ein Detektieren von diskreten Messpunkten bzw. -orten, vorzugsweise von diskreten Sektoren. Vorzugsweise umfasst also jeder Sektor jeweils wenigstens einen Messpunkt.

Die Auflösung des punktweisen Abtastens kann unterschiedlich hoch gewählt werden. Vorzugsweise richtet sich die Auflösung nach der Größe der Sektoren. Für Sektoren, die weniger als 2 Grad Gesichtswinkel von der Fovea beabstandet sind, kann die Auflösung bei 0,05° bis 0,15° Gesichtswinkel, vorzugsweise bei 0,08° bis 0,12° Gesichtswinkel, besonders bevorzugt bei 0,1° Gesichtswinkel liegen. Für periphere Sektoren, die mehr als 20 Grad Gesichtswinkel von der Fovea beabstandet sind, kann die Auflösung bei 1° bis 3° Gesichtswinkel, vorzugsweise bei 1,5° bis 2,5° Gesichtswinkel, besonders bevorzugt bei 2° Gesichtswinkel liegen.

Zur Bestimmung der äußeren Grenzlinie werden vorzugsweise zunächst die einen Rand des Skotoms bildenden Randsektoren bestimmt, das heißt Sektoren, die selbst keine subjektive Sichtbarkeit P für die Person aufweisen und zu Sektoren sowohl mit als auch ohne subjektive Sichtbarkeit P für die Person benachbart sind. Die äußere Grenzlinie kann beispielsweise durch ein Bestimmen der Mittelpunkte dieser Randsektoren, ein Bestimmen von jeweils benachbarten Randsektoren und durch stückweises Verbinden der Mittelpunkte von jeweils benachbarten Randsektoren mit Geradenstücken bestimmt werden. Die äußere Grenzlinie kann stattdessen auch beispielsweise durch Berechnen einer Fitkurve zu den Mittelpunkten der Randsektoren bestimmt werden. Alternativ dazu kann die äußere Grenzlinie als die Randsektoren an der Außenseite des Skotoms unmittelbar umgebende Linie bestimmt werden. Vorzugsweise werden die vorstehend genannten Bestimmungs-, Verbindungs- und/oder Berechnungsschritte durch einen Algorithmus durchgeführt.

Der vorbestimmte Abstand liegt vorzugsweise zwischen 0,3° und 0,8° Gesichtswinkel, weiter bevorzugt zwischen 0,4° und 0,6° Gesichtswinkel. Als besonders vorteilhaft hat sich ein vorbestimmter Abstand von 0,5° Gesichtswinkel erwiesen. Bei der Festlegung des vorbestimmten Abstands ist es vorteilhaft, zwei Kriterien zu berücksichtigen. Zum einen sollte die äußere Hüllkurve möglichst nahe am Skotom liegen, zum anderen sollte die äußere Hüllkurve im Bereich einer vollständig intakten Netzhaut liegen.

Die äußere Hüllkurve kann beispielsweise durch binäre Dilatation in einem vorbestimmten Abstand von der äußeren Grenzlinie erzeugt werden. Als strukturierendes Element kann dabei beispielswiese ein Kreis verwendet werden.

Das Anzeigen der äußeren Hüllkurve als Umrahmung des Skotoms stellt ein präzises und intuitives Feedback bezüglich der Größe und Form des Skotoms für die Person bereit.

Nach einer bevorzugten Ausführungsform der Erfindung umfasst das Verfahren den weiteren Schritt:
Bestimmen einer Position eines bevorzugten retinalen Fixationsortes.

Dazu hat die Person einen auf der Anzeigeeinheit angezeigten Fixierpunkt mit einem (bzw. beiden) Augen zu fixieren. Bei einer ein Zentralskotom aufweisenden Person erfolgt die Fixierung nicht - wie etwa bei einer gesunden Person - mittels der Fovea, sondern mittels des bevorzugten retinalen Fixationsorts als Fovea-Ersatz. Die Position des bevorzugten retinalen Fixationsortes wird vorzugsweise durch eine Auswertung der Augenausrichtung während der Fixierung des Fixierpunkts bestimmt. Vorzugsweise wird die - während die Person den Fixierpunkt fixieren soll - hauptsächlich vorherrschende Augenausrichtung als Position des bevorzugten retinalen Fixationsortes angenommen. Bei manchen Personen sind mehrere bevorzugte retinale Fixationsorte ausgebildet. Die mehreren bevorzugten retinalen Fixationsorte können beispielsweise jeweils für spezifische Sehaufgaben ausgebildet sein. Entsprechend kann vorgesehen sein, dass das Vorhandensein von mehreren bevorzugten retinalen Fixationsorten erfasst wird und deren jeweilige Position bestimmt wird.

Nach einer weiteren bevorzugten Ausführungsform der Erfindung ist vorgesehen, dass das punktweise Abtasten des Gesichtsfelds der Person erfolgt, während die Person einen dauerhaft auf der Anzeigeeinheit angezeigten Fixierpunkt mit dem bevorzugten retinalen Fixationsort zu fixieren hat. Vorzugsweise erfolgt das punktweise Abtasten durch einen zeitweise auf der Anzeigeeinheit angezeigten Abtastpunkt, und werden beim punktweisen Abtasten durch den Abtastpunkt sukzessive einzelne Punkte des Gesichtsfelds in Polarkoordinaten mit Abstand ρ und Winkel θ bezüglich des bevorzugten retinalen Fixationsortes erfasst.

Diese Ausgestaltung ist einfach umzusetzen, bedeutet keinen apparativen Mehraufwand und liefert präzise Ergebnisse.

Die Begriffe "Fixierpunkt" und "Abtastpunkt" sind im Sinne der Erfindung keineswegs nur auf einen einzigen Punkt einschränkt zu verstehen.

Der Abtastpunkt kann ein beliebig geformtes auf der Anzeigeeinheit anzeigbares Abtastobjekt sein. Der Abtastpunkt kann ein abstraktes geometrisches Objekt sein, beispielsweise ein Punkt, ein Kreis, eine Ellipse, ein Rechteck, ein Quadrat oder ein beliebiges anderes Polygon. Dieses Objekt kann ausgefüllt sein oder nur eine Außenlinie aufweisen. Beispielsweise kann der Abtastpunkt eine Kreisscheibe oder ein Kreisring sein. Der Abtastpunkt kann einfarbig, insbesondere schwarz oder weiß, oder mehrfarbig sein. Insbesondere kann der Abtastpunkt ein konkretes Objekt sein, beispielsweise ein Stern, ein Fußball, eine Blüte, eine Katze oder ein Mensch. Das konkrete Objekt kann gezeichnet oder skizziert sein. Das konkrete Objekt kann aber auch fotorealistisch sein. Der Abtastpunkt kann animiert sein, also eine Bewegung ausführen, vorzugsweise eine ortsfeste Bewegung. Beispielsweise kann der Abtastpunkt eine Drehbewegung oder eine pulsierende Bewegung ausführen.

Der Fixierpunkt kann ein beliebig geformtes auf der Anzeigeeinheit anzeigbares Fixierobjekt sein. Der Fixierpunkt kann ein abstraktes geometrisches Objekt sein, beispielsweise ein Punkt, ein Kreis, eine Ellipse, ein Rechteck, ein Quadrat oder ein beliebiges anderes Polygon. Dieses Objekt kann ausgefüllt sein oder nur eine Außenlinie aufweisen. Beispielsweise kann der Fixierpunkt ein Kreuz sein. Der Fixierpunkt kann einfarbig, insbesondere schwarz oder weiß, oder mehrfarbig sein. Insbesondere kann der Fixierpunkt ein konkretes Objekt sein, beispielsweise ein Stern, ein Fußball, eine Blüte, eine Katze oder ein Mensch. Das konkrete Objekt kann gezeichnet oder skizziert sein. Das konkrete Objekt kann aber auch fotorealistisch sein. Der Fixierpunkt kann animiert sein, also eine Bewegung ausführen, vorzugsweise eine ortsfeste Bewegung. Beispielsweise kann der Fixierpunkt eine Drehbewegung oder eine pulsierende Bewegung ausführen.

Vorzugsweise unterscheidet sich der Abtastpunkt vom Fixierpunkt in Form, Größe und/oder Farbe. Vorzugsweise sind der Abtastpunkt und der Fixierpunkt verschiedenartige Objekte.

Der Abtastpunkt wird vorzugsweise für eine vorgegebene Zeitdauer an einem vorgegebenen Ort auf der Anzeigeeinheit angezeigt und erlischt nach Ablauf dieser vorgegebenen Zeitdauer wieder. Anschließend wird der Abtastpunkt jeweils für die vorgegebene Zeitdauer an einem jeweils anderen vorgegebenen Ort auf der Anzeigeeinheit angezeigt und erlischt jeweils nach Ablauf der vorgegebenen Zeitdauer wieder.

Wie oben bereits beschrieben wird das Gesichtsfeld für das punktweise Abtasten vorzugsweise in einzelne Sektoren eines Polarkoordinatensystems aufgeteilt. Vorzugsweise wird der Abtastpunkt in einem gegebenen Zeitpunkt einem vorgegebenen Sektor zugeordnet und auf der Anzeigeeinheit für eine vorgegebene Zeitdauer derart angezeigt, dass das Gesichtsfeld der Person in einem diesen Sektor entsprechenden Bereich bei einer Fixierung des Auges oder der Augen auf den Fixierpunkt mit dem Abtastpunkt beaufschlagt wird. Nach Ablauf der vorgegebenen Zeitdauer erlischt der Abtastpunkt auf der Anzeigeeinheit. Zu einem darauffolgenden Zeitpunkt wird der Abtastpunkt einem anderen Sektor zugeordnet und auf der Anzeigeeinheit für die vorgegebene Zeitdauer derart angezeigt, dass das Gesichtsfeld der Person in einem diesem anderen Sektor entsprechenden Bereich bei einer Fixierung des Auges oder der Augen auf den Fixierpunkt mit dem Abtastpunkt beaufschlagt wird. Auf diese Weise durchläuft der Abtastpunkt sukzessive die einzelnen Sektoren. Es kann vorgesehen sein, dass der Abtastpunkt alle Sektoren des Gesichtsfelds oder nur bestimmte Sektoren erreicht, beispielsweise nur alle Sektoren im zentralen Bereich, insbesondere vorzugsweise nur Sektoren mit einem Abstand von höchstens 5° Gesichtswinkel vom bevorzugten retinalen Fixationsort. Vorzugsweise orientiert sich die Größe des Abtastpunkts an der Größe des jeweiligen Sektors. Solange eine Zuordnung zu einem bestimmten Sektor gegeben ist, kann der Abtastpunkt auch größer sein als der Sektor.

Die Größe des Abtastpunkts kann variieren, insbesondere in Abhängigkeit von seiner Position im Gesichtsfeld. Beispielsweise sind bei einer Darstellung des Gesichtsfelds in Polarkoordinaten Sektoren im peripheren Bereich des Gesichtsfelds größer als Sektoren im zentralen Bereich des Gesichtsfelds. Der Abtastpunkt kann entsprechend für eine Position im peripheren Bereich des Gesichtsfelds größer sein als für eine Position im zentralen Bereich des Gesichtsfelds.

Der Abtastpunkt und/oder der Fixierpunkt können in ein Muster eingebettet sein oder Teil des Musters sein. Das Muster kann beispielsweise ein Radialfrequenzmuster, engl. radial frequency pattern, sein. Der Abtastpunkt und/oder der Fixierpunkt können in ein gezeichnetes oder fotorealistisches Bild eingebettet sein oder Teil des Bilds sein. Das Bild kann beispielsweise ein Waldo-Bild sein. Das Bild kann ein Standbild oder ein animiertes Bild sein. Der Abtastpunkt und/oder der Fixierpunkt können in eine Filmsequenz eingebettet sein oder in der Filmsequenz erscheinen und/oder verschwinden. Weiterhin kann der Abtastpunkt in unterschiedlicher räumlicher Tiefe in ein Bild (Standbild, animiertes Bild oder Filmsequenz) eingebettet sein. Weiterhin können der Abtastpunkt und/oder der Fixierpunkt in ein Szenario einer virtuellen Realität oder einer erweiterten Realität eingebettet sein oder Teil dieser sein.

Im Fall von mehreren bevorzugten retinalen Fixationsorten kann das punktweise Abtasten jeweils einzeln für die einzelnen bevorzugten retinalen Fixationsorte durchgeführt werden. Alternativ dazu kann die Lagebeziehung der einzelnen bevorzugten retinalen Fixationsorte (also deren Abstand ρ und der jeweilige Winkel θ) bestimmt werden und auf dieser Grundlage ein gemeinsames Abtasten für die mehreren bevorzugten retinalen Fixationsorte durchgeführt werden.

Die Person kann durch ein zusätzliches Signal auf die Anzeige des Abtastpunkts auf der Anzeigeeinheit hingewiesen werden. Das zusätzliche Signal kann ein Audiosignal und/oder ein haptisches Signal sein. Das zusätzliche Signal kann genau während der gesamten Anzeigedauer des Abtastpunkts auf die Anzeige des Abtastpunkts auf der Anzeigeeinheit hinweisen und erst beim Erlöschen des Abtastpunkts enden.

Nach einer weiteren bevorzugten Ausführungsform der Erfindung ist vorgesehen, dass für jeden Punkt (ρ, θ) die subjektive Sichtbarkeit P durch die Person von dieser Person abgefragt wird. Dabei wird zu n derart erfassten Punkten (ρ, θ) eine 3xn-Matrix mit Einträgen (ρ, θ, P) und/oder als graphische Darstellung der n erfassten Punkten (ρ, θ) eine Gesichtsfeldkarte gebildet.

Die Abfrage der subjektiven Sichtbarkeit P kann erfolgen, indem die Person einen Knopf oder mehrere Knöpfe zum Bedienen erhält und durch entsprechenden Knopfdruck bzw. durch Unterlassen des Knopfdrückens mitteilt, ob der Abtastpunkt zu sehen war bzw. ob der Abtastpunkt nicht zu sehen war. Weiterhin kann die Abfrage der subjektiven Sichtbarkeit P mittels Spracherkennung erfolgen. Dabei hat die Person beispielsweise "Ja" zu sprechen, falls der Abtastpunkt zu sehen war, und zu schweigen, falls der Abtastpunkt nicht zu sehen war. Weiterhin kann die Person instruiert sein, durch ein Blinzeln, vorzugsweise durch doppeltes Blinzeln, die subjektive Sichtbarkeit P zu signalisieren. Entsprechend kann die Abfrage der subjektiven Sichtbarkeit P durch Detektieren der Bewegung der Augenlider erfolgen.

Alternativ oder ergänzend kann die subjektive Sichtbarkeit P auch auf der Grundlage einer Reaktion der Pupille des Auges bestimmt werden.

Die subjektive Sichtbarkeit P kann beispielsweise den Wert 1 für den Fall annehmen, dass der Abtastpunkt zu sehen war, und den Wert 0 annehmen, falls der Abtastpunkt nicht zu sehen war.

Auf der Gesichtsfeldkarte können die verschiedenen Werte von P in verschiedenen Farben oder mit verschiedenen Symbolen dargestellt werden.

Mit Hilfe der Gesichtsfeldkarte kann besonders schnell und effektiv die Form und Größe eines Skotoms bestimmt werden.

Zur Bestimmung der Form und Größe des Skotoms können Interpolationsmethoden und maschinelles Lernen eingesetzt werden.

Die erfindungsgemäß berechnete äußere Grenzlinie des Skotoms kann durch eine Überprüfung durch die Person verifiziert werden. Bei dieser Überprüfung wird der Person die äußere Hüllkurve erfindungsgemäß als Umrahmung des Skotoms auf der Anzeigeeinheit angezeigt.

Durch eine Überprüfung diskreter Skotombereiche können feinere Anpassungen der äußeren Grenzlinie und/oder der äußeren Hüllkurve vorgenommen werden.

Nach einer weiteren bevorzugten Ausführungsform der Erfindung ist vorgesehen, dass als Maß für die Qualität der Fixierung des auf der Anzeigeeinheit angezeigten Fixierpunkts durch den bevorzugten retinalen Fixationsort eine Fixationsstabilität aus der fortwährend erfassten Augenausrichtung bestimmt wird.

Dabei ist möglich, dass ein Grenzwert für die Fixationsstabilität vorgegeben wird. Das Verfahren kann so ausgestaltet sein, dass die subjektive Sichtbarkeit P zu einem Abtastpunkt verworfen wird, falls die während der Anzeige dieses Abtastpunkts ermittelte Fixationsstabilität diesen Grenzwert unterschreitet.

Nach einer weiteren bevorzugten Ausführungsform der Erfindung ist vorgesehen, dass eine Korrektur der Polarkoordinaten eines Punkts (ρ, θ) des Gesichtsfelds durch die fortwährend erfasste Augenausrichtung erfolgt, falls der Fixierpunkt im Moment des Abtastens durch den Abtastpunkt nicht mit dem bevorzugten retinalen Fixationsort fixiert ist.

Durch eine Auswertung der fortwährend erfassten Augenausrichtung kann eine Lagebeziehung zwischen dem tatsächlichen Fixationsort während der Anzeige des Abtastpunkts und dem bevorzugten retinalen Fixationsort ermittelt werden. Die Korrektur kann auf der Grundlage dieser Lagebeziehung durchgeführt werden.

Die Korrektur kann in Abhängigkeit von der Fixationsstabilität durchgeführt werden. Es kann beispielsweise ein weiterer Grenzwert für die Fixationsstabilität vorgegeben werden. Das Verfahren kann so ausgestaltet sein, dass die Korrektur durchgeführt wird, falls die während der Anzeige des Abtastpunkts ermittelte Fixationsstabilität diesen weiteren Grenzwert unterschreitet.

Das punktweise Abtasten des Gesichtsfelds mit der vorgenannten Korrektur der Polarkoordinaten (ρ, θ) des Abtastpunkts mittels der fortwährend erfassten Augenausrichtung führt zu besonders zuverlässigen Ergebnissen.

Nach einer weiteren bevorzugten Ausführungsform der Erfindung ist vorgesehen, dass zusätzlich ein Bild vom Augenhintergrund des Auges der Person aufgenommen wird. Dabei wird auf der Grundlage des Bilds die Position der Fovea bestimmt. Das Bild und/oder die Position der Fovea werden gespeichert.

Für die Aufnahme des Bilds vom Augenhintergrund können verschiedene bildgebende Verfahren verwendet werden, beispielsweise die Scanning-Laser-Ophthalmoskopie (SLO), die optische Kohärenztomografie (*engl. optical coherence tomography,* OCT) oder die Fundusfotografie. Insbesondere kann die Lagebeziehung zwischen der Fovea und dem bevorzugten retinalen Fixationsort auf der Grundlage des Bilds vom Augenhintergrund sowie der Augenausrichtung beim Fixieren eines Fixierpunkts mittels des bevorzugten retinalen Fixationsorts ermittelt werden.

Nach einer weiteren bevorzugten Ausführungsform der Erfindung ist vorgesehen, dass ein für eine spezifische Sehaufgabe optimaler Bereich aus den sehtauglichen Punkten im Gesichtsfeld der Person als Ersatz für den bevorzugten retinalen Fixationsort bestimmt wird.

Der für eine spezifische Sehaufgabe optimale Bereich wird hier im Weiteren als optimaler bevorzugter retinaler Fixationsort bezeichnet. Der optimale bevorzugte retinale Fixationsort ist ein für eine spezifische Sehaufgabe besonders effizienter Ersatzort für den bevorzugten retinalen Fixationsort.

Der optimale bevorzugte retinale Fixationsort kann beispielsweise in Abhängigkeit von der Form und Größe des Skotoms, insbesondere vom Verlauf der äußeren Grenzlinie des Skotoms und/oder in Abhängigkeit der zuvor bestimmten sehuntauglichen Punkte im Gesichtsfeld der Person bestimmt werden.

Nach einer weiteren bevorzugten Ausführungsform der Erfindung ist vorgesehen, dass die fortwährend erfasste Augenausrichtung, die sehtauglichen Punkte im Gesichtsfeld der Person, die sehuntauglichen Punkte im Gesichtsfeld der Person, die äußere Grenzlinie des Skotoms, die äußere Hüllkurve, die Position des bevorzugten retinalen Fixationsortes, die Position der Fovea und/oder der für eine spezifische Sehaufgabe optimale Bereich mit einem Zeitpunkt der jeweiligen Bestimmung für das Auge oder die Augen der Person als aktuelle Daten der Person gespeichert werden, und
wobei aus den aktuellen Daten der Person, aus früheren entsprechend bestimmten Daten der Person und/oder aus entsprechend bestimmten Daten anderer Personen, vorzugsweise mittels künstlicher Intelligenz, Aussagen für die weitere Entwicklung des Skotoms der Person, für die Position des
bevorzugten retinalen Fixationsortes der Person und/oder für den für eine spezifische Sehaufgabe optimalen Bereich der Person erzeugt werden.

Bei einem Vorhandensein mehrerer bevorzugter retinaler Fixationsorte wird die Position jedes der bevorzugten retinalen Fixationsorte gespeichert. In diesem Fall können Aussagen für die jeweiligen Positionen der mehreren bevorzugten retinalen Fixationsorte der Person erzeugt werden.

Die bevorzugte Ausführungsform der Erfindung erlaubt insbesondere die subjektive Messung der Größe und Form des Skotoms einer bestimmten Person als Funktion der Zeit.

Weiterhin können aus den aktuellen Daten der Person, aus früheren entsprechend bestimmten Daten der Person und/oder aus entsprechend bestimmten Daten anderer Personen spezifische Maßnahmen für die Person bestimmt werden. Beispielsweise können eine für die Person lesbare Mindestschriftgröße, ein Trainingsplan und/oder der Zeitpunkt des nächsten Untersuchungstermins bestimmt werden.

Die bevorzugte Ausführungsform der Erfindung erlaubt detaillierte Vorhersagen bzw. Prognosen über die Entwicklung des Skotoms und der Erfolgsaussichten des Trainings.

Nach einer weiteren bevorzugten Ausführungsform der Erfindung ist vorgesehen, dass ein Training des bevorzugten retinalen Fixationsortes der Person mittels der Anzeigeeinheit kombiniert mit dem Anzeigen der äußeren Hüllkurve auf der Anzeigeeinheit durchgeführt wird.

Das Training des bevorzugten retinalen Fixationsortes wird mit dem Ziel durchgeführt, die Position des bevorzugten retinalen Fixationsorts in den für eine spezifische Sehaufgabe optimalen Bereich zu verlagern. Die äußere Hüllkurve wird erfindungsgemäß auf der Anzeigeeinheit unter Berücksichtigung der fortwährend erfassten Augenausrichtung derart angezeigt, dass die äußere Hüllkurve für die Person bei beliebiger Ausrichtung des Auges der Person auf die Anzeigeeinheit als Umrahmung des Skotoms wahrnehmbar ist. Die äußere Hüllkurve wird vorzugsweise fortwährend während des Trainings auf der Anzeigeeinheit angezeigt. Das Training kann besonders bevorzugt durchgeführt werden, indem zusätzlich ein Szenario einer virtuellen Realität oder erweiterten Realität auf der Anzeigeeinheit angezeigt wird. Es hat sich gezeigt, dass bei einer Kombination eines Szenarios einer virtuellen Realität oder erweiterten Realität mit der fortwährenden Anzeige der äußeren Hüllkurve als Umrahmung des Skotoms das Training zur Verlagerung des bevorzugten retinalen Fixationsorts zum optimalen bevorzugten retinalen Fixationsort für die spezifische Sehaufgabe besonders effektiv ist.

Vorteilhafterweise kann auch ein Training für spezifische Bereiche des Gesichtsfelds bei Personen ohne Skotom erfolgen. Dabei kann ein spezifisches Blickverhalten trainiert werden, beispielsweise das Blickverhalten bei der Verwendung von Gleitsichtgläsern.

Die erfindungsgemäße Vorrichtung zum Erfassen eines Gesichtsfelds einer ein Skotom, insbesondere ein Zentralskotom, aufweisenden Person, umfasst eine Erfassungseinheit zum fortwährenden Erfassen der Augenausrichtung der Person, eine Anzeigeeinheit zum Anzeigen von visueller Information, wobei die Anzeigeeinheit in einer definierten und/oder bestimmbaren räumlichen Beziehung zur Position des Auges der Person angeordnet ist, und eine Steuereinheit zum Durchführen des erfindungsgemäßen Verfahrens und/oder von beliebigen oben beschriebenen Ausführungsformen davon.

Das punktweise Abtasten des Gesichtsfelds der Person wird vorzugsweise mittels der Anzeigeeinheit durchgeführt. Die räumliche Beziehung der Anzeigeeinheit zur Position des Auges der Person kann fest vorgegeben sein. Alternativ kann eine Bewegung der Position des Auges bzw. des Kopfs der Person relativ zur Anzeigeeinheit erlaubt und durch Mittel zur Bestimmung der Position der Augen bzw. des Kopfs bestimmbar sein.

Die Erfassungseinheit ist vorzugsweise ein Gerät zur Blickerfassung, insbesondere ein sog. Eye-Tracker. Die Erfassungseinheit umfasst vorzugsweise eine auf das Auge oder auf beide Augen der Person ausrichtbare Kamera zur fortwährenden Aufnahme von Bildern des Auges oder der Augen. Die Erfassungseinheit kann zusätzlich eine Auswerteeinheit zum Auswerten der Bilder und zum fortwährenden Berechnen der Augenausrichtung der Person umfassen. Alternativ kann vorgesehen sein, dass durch die Kamera aufgenommene Bilder fortwährend zur Steuereinheit übertragen werden und dass die Steuereinheit die Augenausrichtung der Person fortwährend berechnet. Die Erfassungseinheit kann weiterhin die Mittel zur Bestimmung der Position der Augen bzw. des Kopfs umfassen. In diesem Fall bestimmt die Erfassungseinheit zusätzlich zur Augenausrichtung die Position der Augen bzw. des Kopfs der Person.

Die erfindungsgemäße Vorrichtung kann zusätzlich eine Feedbackeinheit zur Erfassung der subjektiven Sichtbarkeit P eines Punkts (ρ, θ) durch die Person umfassen. Die Feedbackeinheit kann eine Betätigungseinheit mit einem oder mehreren Knöpfen, die die Person zur Mitteilung der subjektiven Sichtbarkeit P des Punkts (ρ, θ) zu betätigen hat, sein. Die Feedbackeinheit kann weiterhin eine Spracherkennungseinheit zum Erfassen und zum Verarbeiten einer Sprachantwort der Person sein. Die Feedbackeinheit kann auch als Augenliderdetektionseinheit zum Detektieren der Bewegung der Augenlider ausgestaltet sein, wobei die Person instruiert ist, durch ein Blinzeln, vorzugsweise durch doppeltes Blinzeln, die subjektive Sichtbarkeit P zu signalisieren. Alternativ oder ergänzend kann die Feedbackeinheit auch eine Pupillenreaktionsdetektionseinheit zum Detektieren einer Reaktion der Pupille des Auges und Bestimmen der subjektiven Sichtbarkeit P umfassen.

Die erfindungsgemäße Vorrichtung und/oder die Steuereinheit kann zusätzlich eine Speichereinheit zum Speichern der fortwährend erfassten Augenausrichtung, der sehtauglichen Punkte im Gesichtsfeld, der sehuntauglichen Punkte im Gesichtsfeld, der äußeren Grenzlinie des Skotoms, der äußeren Hüllkurve, der Position des bevorzugten retinalen Fixationsortes, der Position der Fovea und/oder des für eine spezifische Sehaufgabe optimalen Bereichs zusammen mit einem Zeitpunkt der jeweiligen Bestimmung und mit einer Zuordnung zur jeweiligen Person umfassen. In der Speichereinheit können also insbesondere aktuelle Daten der Person, frühere entsprechend bestimmte Daten der Person und/oder entsprechend bestimmte Daten anderer Personen gespeichert sein.

Die erfindungsgemäße Vorrichtung und/oder die Steuereinheit kann zusätzlich eine Vorhersageeinheit zur Erzeugung von Aussagen für die weitere Entwicklung des Skotoms der Person, für die Position des bevorzugten retinalen Fixationsortes der Person und/oder für den für eine spezifische Sehaufgabe optimalen Bereich der Person, vorzugsweise mittels künstlicher Intelligenz, aus den aktuellen Daten der Person, aus früheren entsprechend bestimmten Daten der Person und/oder aus entsprechend bestimmten Daten anderer Personen umfassen.

Nach einer bevorzugten Ausführungsform der Erfindung ist vorgesehen, dass die Anzeigeeinheit ein Bildschirm, insbesondere ein sphärisch geformter Bildschirm, ist, und dass die Vorrichtung weiterhin Mittel zur Platzierung des Kopfs der Person in einer relativ zum Bildschirm definierten Position und/oder Mittel zur Bestimmung einer Position des Kopfs relativ zum Bildschirm umfasst.

Dabei kann insbesondere eine Kinnstütze als Mittel zur Platzierung des Kopfs der Person in einer relativ zum Bildschirm definierten Position vorgesehen sein.

Die Mittel zur Bestimmung einer Position des Kopfs relativ zum Bildschirm können durch die Erfassungseinheit bereitgestellt werden. In diesem Fall werden auf der Grundlage der durch die Kamera fortwährend aufgenommen Bilder durch die Auswerteeinheit oder durch die Steuereinheit zusätzlich zur Augenausrichtung auch die Kopfposition und/oder die Augenposition der Person fortwährend berechnet.

Alternativ dazu können die Mittel zur Bestimmung einer Position des Kopfs relativ zum Bildschirm durch eine separate Positionsbestimmungseinheit bereitgestellt werden. Die Positionsbestimmungseinheit kann ein weiterer Eye-Tracker sein. Die Positionsbestimmungseinheit umfasst vorzugsweise eine auf den Kopf oder das Auge bzw. die Augen der Person ausrichtbare Kamera zur fortwährenden Aufnahme von Bildern des Kopfs oder des Auges bzw. der Augen. Aus den durch die Kamera fortwährend aufgenommenen Bildern wird durch die Positionsbestimmungseinheit oder durch die Steuereinheit die Kopfposition und/oder die Augenposition der Person fortwährend berechnet. Die Mittel zur Bestimmung einer Position des Kopfs relativ zum Bildschirm ermöglichen eine freie Bewegung der Person in einem definierten Raumbereich. Eine auf einem Stuhl sitzende Person darf sich bei dieser Ausführungsform beispielsweise frei bewegen.

Nach einer alternativen bevorzugten Ausführungsform der Erfindung ist vorgesehen, dass die Anzeigeeinheit und die Erfassungseinheit in einer am Kopf der Person befestigten Kopfeinheit vorgesehen sind.

Die am Kopf befestigte Einheit wird nachfolgend wie allgemein üblich als Head-Mounted Display bezeichnet. Die Steuereinheit kann in das Head-Mounted Display integriert oder als eigenständiges Bauteil am Körper der Person ausgebildet oder ortsfest, also unabhängig von der Bewegung der Person, angeordnet sein. Es kann eine Datenverbindung zwischen der Steuereinheit und dem Head-Mounted Display vorgesehen sein. Die Datenverbindung kann kabelgebunden oder als Funkverbindung (W-Lan, Bluetooth etc.) ausgestaltet sein. Das Head-Mounted Display kann weiterhin einen und mehrere Lagesensoren umfassen, insbesondere ein Magnetometer, einen Beschleunigungssensor und/oder ein Gyroskop. Ein am Körper der Person, beispielsweise in einer Hosentasche getragenes Smartphone kann als Steuereinheit fungieren. Weiterhin können die Steuereinheit, die Anzeigeeinheit und/oder die Erfassungseinheit durch ein mit einer speziellen Halterung als Head-Mounted Smartphone genutztes Smartphone realisiert sein.

Die Ausgestaltung der Anzeigeeinheit und der Erfassungseinheit als Bestandteile eines Head-Mounted Display ist besonders vorteilhaft zur Anzeige einer virtuellen Realität oder einer erweiterten Realität. Es hat sich dabei gezeigt, dass die Kombination eines Szenarios einer virtuellen Realität oder einer erweiterten Realität mit der Anzeige der äußeren Hüllkurve als Umrahmung des Skotoms auf dem Head-Mounted Display das Training des bevorzugten retinalen Fixationsorts besonders effektiv macht.

Die erfindungsgemäße Vorrichtung oder vorstehend beschriebene vorteilhafte Ausgestaltungen davon können weitere Trainingsarten ermöglichen, beispielsweise das Training für spezifische Bereiche des Gesichtsfelds bei Personen ohne Skotom. Dabei kann ein spezifisches Blickverhalten trainiert werden, beispielsweise das Blickverhalten bei der Verwendung von Gleitsichtgläsern.

Das erfindungsgemäße Verfahren und/oder beliebige oben beschriebene bevorzugte Ausführungsformen davon können computerimplementiert sein. Erfindungsgemäß ist insbesondere ein Computerprogramm mit auf einem Prozessor ablauffähigem Programmcode zum Ausführen der Verfahrensschritte des erfindungsgemäßen Verfahrens und/oder von beliebigen oben beschriebenen Ausführungsformen davon vorgesehen. Insbesondere führt das Computerprogramm die Verfahrensschritte des erfindungsgemäßen Verfahrens aus, indem es einen Algorithmus durchläuft.

Das Computerprogramm wird dazu auf einem Computer geladen oder auf einem Computer ausgeführt. Die Steuereinheit kann als Computer ausgestaltet sein oder einen Computer umfassen. Alternativ kann der Computer die Steuereinheit umfassen.

Erfindungsgemäß ist weiterhin ein nichttransitorisches Speichermedium mit einem darauf gespeicherten erfindungsgemäßen Computerprogramm vorgesehen.

Die Erfindung ermöglicht vorteilhafterweise eine unmittelbare und präzise Bestimmung der Größe, der Form und der Entwicklung eines Skotoms, insbesondere Zentralskotoms. Die Verwendung eines Szenarios einer virtuellen Realität oder einer erweiterten Realität trägt besonders zur Motivation des Patienten bei. Ein solches Szenario beansprucht den Patienten und verhindert ein Abstumpfen des Patienten durch Langeweile. Besonders vorteilhaft ist die Kombination eines Szenarios einer virtuellen Realität oder einer erweiterten Realität mit der Anzeige der äußeren Hüllkurve als Umrahmung des Skotoms insbesondere auf einem Head-Mounted Display als Anzeigeeinheit. Vorteilhafterweise wird ein optimaler bevorzugter retinaler Fixationsort in Abhängigkeit von der Form des Skotoms und in Abhängigkeit der spezifischen Sehaufgabe bestimmt. Es hat sich gezeigt, dass dabei das Training zur Verlagerung des bevorzugten retinalen Fixationsorts zum optimalen bevorzugten retinalen Fixationsort für die spezifische Sehaufgabe besonders effektiv ist.

Die Erfindung wird nun anhand von Ausführungsbeispielen näher erläutert. Es zeigen
- Fig. 1: ein Ablaufschema beim punktweisen Abtasten des Gesichtsfelds,
- Fig. 2: eine Gesichtsfeldkarte als graphische Darstellung der subjektiven Sichtbarkeit P der n erfassten Punkte (ρ, θ),
- Fig. 3: die Gesichtsfeldkarte mit äußerer Grenzlinie des Skotoms,
- Fig. 4: eine weitere Darstellung der Gesichtsfeldkarte mit zwei zusätzlich eingezeichneten optimalen bevorzugten retinalen Fixationsorten für zwei verschiedene spezifische Sehaufgaben, und
- Fig. 5: eine vergrößerte schematische Darstellung des Skotoms mit äußerer Grenzlinie und äußerer Hüllkurve.

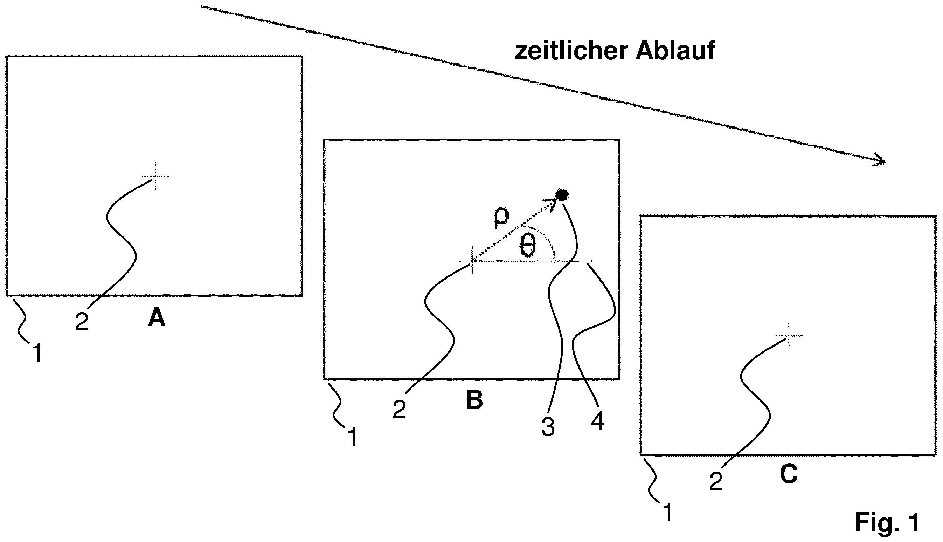

Fig. 1 zeigt ein Ablaufschema beim punktweisen Abtasten des Gesichtsfelds. Dabei ist eine Anzeigeeinheit 1, insbesondere ein Bildschirm, zu drei verschiedenen aufeinanderfolgenden Zeitpunkten A, B und C gezeigt. Eine Person ist instruiert, einen dauerhaft auf der Anzeigeeinheit 1 angezeigten Fixierpunkt 2 mit ihrem linken und/oder rechten Auge zu fixieren. Der Fixierpunkt 2 ist beispielsweise als ein Kreuz ausgestaltet. Zum Zeitpunkt B erscheint zusätzlich ein Abtastpunkt 3 auf der Anzeigeeinheit 1 für eine vorgegebene Zeitdauer. Der Abtastpunkt 3 ist beispielsweise als eine Kreisscheibe ausgestaltet und (dessen Mittelpunkt) weist einen Abstand ρ vom Fixierpunkt 2 und einen Winkel θ bezüglich einer vorzugsweise horizontal durch den Fixierpunkt 2 verlaufenden Linie auf. Der Fixierpunkt 2 definiert also den Ursprung eines Polarkoordinatensystems mit der Linie als Polarachse 4. Der Abtastpunkt 3 wird durch die Polarkoordinaten (ρ, θ) in diesem Polarkoordinatensystem verortet. Zusätzlich zur Anzeige des Abtastpunkts 3 wird die Person durch ein akustisches oder haptisches Signal auf die gleichzeitige Anzeige des Abtastpunkts 3 aufmerksam gemacht. Die Person ist instruiert, über eine Feedbackeinheit (nicht gezeigt) mitzuteilen, ob der Abtastpunkt 3 wahrgenommen wird. Die Feedbackeinheit umfasst beispielsweise einen bei Wahrnehmung des Abtastpunkts 3 zu drückenden Knopf. Nach Ablauf der vorgegebenen Zeitdauer erlischt der Abtastpunkt 3 wieder (Zeitpunkt C). Das akustische oder haptische Signal endet ebenfalls. Vorzugsweise wird ein nach dem Erlöschen des Abtastpunkts 3 erfolgendes Knopfdrücken durch die Feedbackeinheit erfasst und dem erloschenen Abtastpunkt 3 zugeordnet. Nach dem Erfassen des Knopfdrückens durch die Feedbackeinheit und/oder nach Ablauf einer weiteren Zeitdauer erscheint der Abtastpunkt 3 an einem anderen Ort auf der Anzeigeeinheit 1 vorzugsweise wieder für die vorgegebene Zeitdauer (nicht gezeigt). Die Anzeige ist wiederum begleitet durch das akustische oder haptische Signal. Nach Ablauf der vorgegebenen Zeitdauer erlischt der Abtastpunkt 3 wieder (nicht gezeigt). Diese Abfolge wird für insgesamt n verschiedene Orte auf der Anzeigeeinheit 1 durchgeführt. Bei dieser Vorgehensweise wird also zu n Paaren von Polarkoordinaten (ρ, θ) jeweils die subjektive Sichtbarkeit P durch die Person erfasst. P kann die Werte 1 (Abtastpunkt 3 für die Person wahrnehmbar) oder 0 (Abtastpunkt 3 für die Person nicht wahrnehmbar) annehmen. Die erfassten Daten werden in einer Speichereinheit mit dem Erfassungszeitpunkt spezifisch für die Person gespeichert. Die erfassten Daten können als eine 3xn-Matrix mit zeilenweisen oder spaltenweisen Einträgen (ρ, θ, P) dargestellt werden.

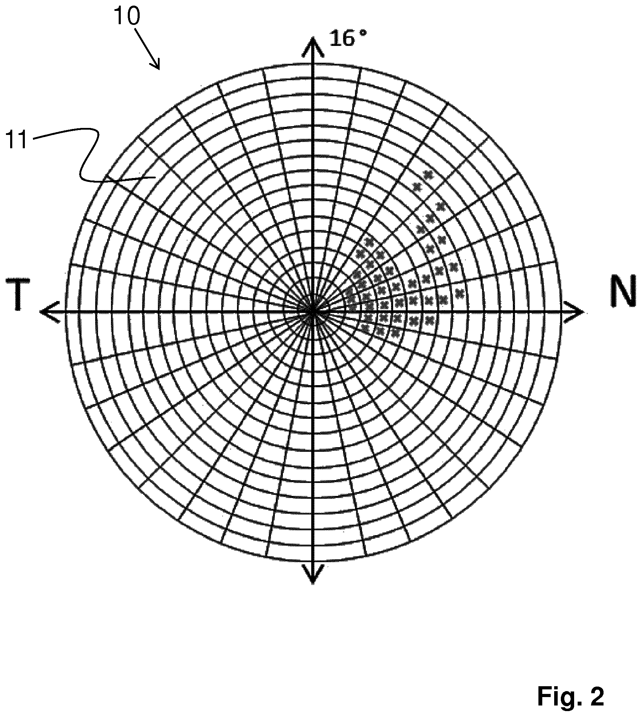

Die erfassten Daten können anschaulicher als Gesichtsfeldkarte dargestellt werden. Fig. 2 zeigt eine Gesichtsfeldkarte 10 als graphische Darstellung der subjektiven Sichtbarkeit P der n erfassten Punkte (ρ, θ). Die Gesichtsfeldkarte 10 ist eine Darstellung des Gesichtsfelds in Polarkoordinaten. Das Gesichtsfeld ist dabei in Polarsektoren 11 unterteilt. Das in Zusammenhang mit Fig. 1 beschriebene punktweise Abtasten des Gesichtsfelds wird derart durchgeführt, dass jeweils ein Abtastpunkt 3 einem Polarsektor 11 der Gesichtsfeldkarte 10 zugeordnet ist. Dabei können alle auf der Gesichtsfeldkarte 10 vorhandenen Polarsektoren 11 jeweils durch einen Abtastpunkt 3 erfasst werden. Vorzugsweise wird jedoch eine Auswahl von n Polarsektoren 11 für das Erfassen der subjektiven Sichtbarkeit P getroffen. Vorzugsweise werden ausschließlich Polarsektoren 11 im zentralen Bereich und/oder in Bereichen, in denen bei früheren Messung bei der Person bereits keine subjektive Sichtbarkeit (P = 0) festgestellt wurde, und dazu benachbarte Bereiche mit subjektiven Sichtbarkeit (P = 1) erfasst. Im Beispiel der Fig. 2 sind alle Polarsektoren 11, bei denen jeweils der Abtastpunkt 3 für die Person nicht wahrnehmbar war (P = 0) mit einem "x" markiert. Alternativ oder zusätzlich können diese Sektoren in einer bestimmten Farbe, z.B. rot, dargestellt sein. In Fig. 2 steht T für temporal, N für nasal. Mit 16° ist ein Referenzwert der Exzentrizität angegeben. Die Messung des Gesichtsfelds kann jedoch auch jenseits von 16° erfolgen.

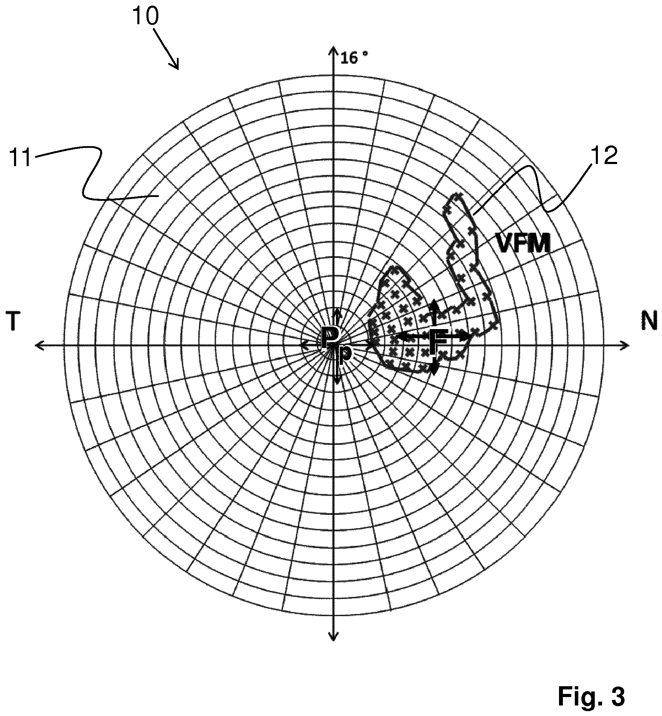

Fig. 3 zeigt die Gesichtsfeldkarte 10 aus Fig. 2, nachdem durch die Steuereinheit aus der Anordnung der Polarsektoren 11 mit P = 0 ein Skotom im Gesichtsfeld der Person aufgefunden wurde und die äußere Grenzlinie 12 des aufgefundenen Skotoms berechnet wurde. Die äußere Grenzlinie 12 ist in Form von Geradenabschnitten in Fig. 3 schematisch eingezeichnet. Die Geradenabschnitte verbinden die Mittelpunkte der einen Rand des Skotoms bildenden Randsektoren. Die äußere Grenzlinie 12 kann jedoch auch als eine durchgehende glatte Linie bestimmt werden. Die äußere Grenzlinie 12 kann in einer bestimmten Farbe, z.B. grün, dargestellt sein. Zusätzlich ist in Fig. 3 die Position des bevorzugten retinalen Fixationsorts Pₚ eingezeichnet. Da die Person den Fixierpunkt 2 mit ihrem bevorzugten retinalen Fixationsort Pₚ fixiert, ist der bevorzugte retinale Fixationsort Pₚ der Ursprung des Polarkoordinatensystems. Zusätzlich kann eine Erfassungseinheit die Augenausrichtung Eye(x,y) der Person fortwährend während des zu Fig. 1 beschriebenen punktweisen Abtastens erfassen. Die so erfasste Augenausrichtung Eye(x,y) kann zusammen mit den oben beschriebenen Daten zu ρ, θ und P, insbesondere mit der 3xn-Matrix, für den Erfassungszeitpunkt spezifisch für die Person gespeichert werden. Die Lage des bevorzugten retinalen Fixationsorts Pₚ bezüglich der Augenachse kann in diesem Fall durch eine Auswertung der durch die Erfassungseinheit während des Fixierens des Fixierpunkts 2 erfassten Augenausrichtung Eye(x,y) bestimmt werden. Zusätzlich können in diesem Fall die Polarkoordinaten (ρ, θ) zu einem Abtastpunkt 3 korrigiert werden, falls die Augenausrichtung Eye(x,y) während der Anzeige des Abtastpunkts 3 nicht dem bevorzugten retinalen Fixationsort Pₚ der Person entspricht. In Fig. 3 ist weiterhin die Position der Fovea F eingezeichnet. Die Position der Fovea F kann durch zusätzliche Aufnahme eines Bilds vom Augenhintergrund des Auges der Person bestimmt werden. Dazu können verschiedene bildgebende Verfahren verwendet werden, beispielsweise die Scanning-Laser-Ophthalmoskopie (SLO), die optische Kohärenztomografie (*engl. optical coherence tomography,* OCT) oder die Fundusfotografie. Die Lagebeziehung zwischen der Fovea F und dem bevorzugten retinalen Fixationsort kann auf der Grundlage des Bilds vom Augenhintergrund sowie der Augenausrichtung Eye(x,y) beim Fixieren des Fixierpunkts 2 mittels des bevorzugten retinalen Fixationsorts Pₚ ermittelt werden.

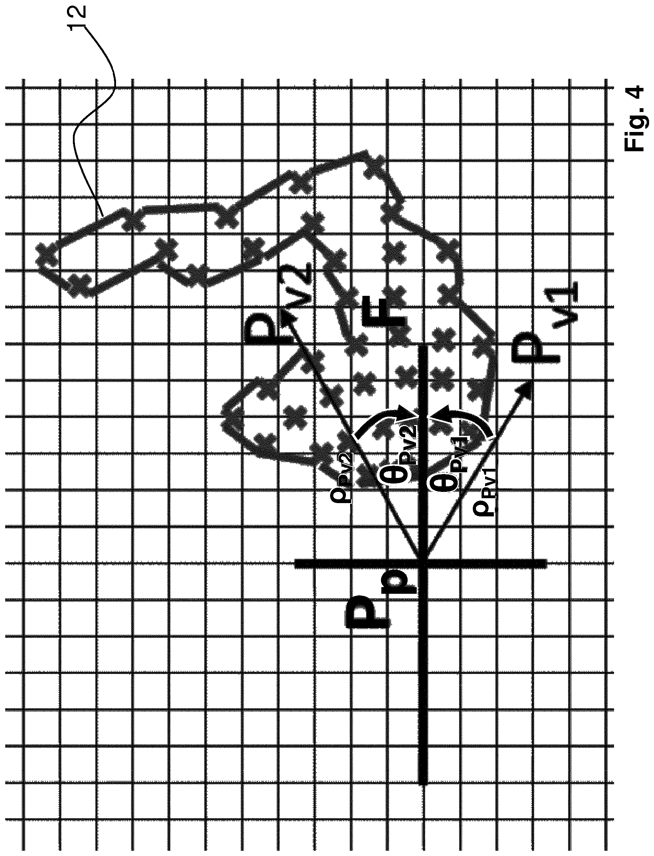

Fig. 4 zeigt eine weitere Darstellung der Gesichtsfeldkarte mit zwei zusätzlich eingezeichneten optimalen bevorzugten retinalen Fixationsorten für zwei verschiedene spezifische Sehaufgaben. Für eine erste spezifische Sehaufgabe v1 kann durch die Steuereinheit ein erster optimaler bevorzugter retinaler Fixationsort Pᵥ₁ berechnet werden. Entsprechend kann für eine zweite spezifische Sehaufgabe v2 durch die Steuereinheit ein zweiter optimaler bevorzugter retinaler Fixationsort Pᵥ₂ berechnet werden. In Fig. 4 sind der erste optimale bevorzugte retinale Fixationsort Pᵥ₁ und der zweite optimale bevorzugte retinale Fixationsort Pᵥ₂ eingezeichnet. Der erste optimale bevorzugte retinale Fixationsort Pᵥ₁ ist beispielsweise für eine Sehaufgabe optimiert, die eine Lage des bevorzugten retinalen Fixationsorts unterhalb des Skotoms benötigt, insbesondere für das Lesen eines Texts. Der zweite optimale bevorzugte retinale Fixationsort Pᵥ₂ ist beispielsweise für eine Sehaufgabe optimiert, die eine Lage des bevorzugten retinalen Fixationsorts in der Nähe der Fovea F benötigt. Die Steuereinheit kann weiterhin erste Translationsvariablen ρ_{Pv1} und θ_{Pv1} für eine Translation des vorhandenen bevorzugten retinalen Fixationsorts Pₚ zum ersten optimalen bevorzugten retinalen Fixationsort Pᵥ₁ und zweite Translationsvariablen ρ_{Pv2} und θ_{Pv2} für eine Translation des vorhandenen bevorzugten retinalen Fixationsorts Pₚ zum zweiten optimalen bevorzugten retinalen Fixationsort Pᵥ₁ berechnen. Die ersten Translationsvariablen (ρ_{Pv1}, θ_{Pv1}) und die zweiten Translationsvariablen (ρ_{Pv2}, θ_{Pv2}) sind ebenfalls in Fig. 4 eingezeichnet.

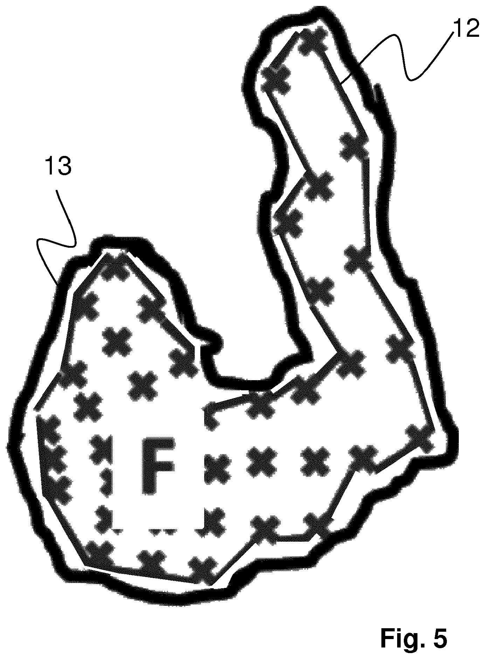

Fig. 5 zeigt eine vergrößerte schematische Darstellung des Skotoms. Die Steuereinheit kann auf der Grundlage der äußeren Grenzlinie 12 eine äußere Hüllkurve 13 berechnen. Die äußere Hüllkurve 13 wird so berechnet, dass sie die äußere Grenzlinie 12 in einem vorbestimmten Abstand umgibt. Der vorbestimmte Abstand liegt vorzugsweise bei etwa 0,5° Gesichtswinkel.

Die äußere Hüllkurve 13 kann auf der Anzeigeeinheit 1 unter Berücksichtigung der fortwährend erfassten Augenausrichtung Eye(x,y) derart angezeigt werden, dass die äußere Hüllkurve 13 für die Person bei beliebiger Ausrichtung des Auges auf die Anzeigeeinheit 1 als Umrahmung des Skotoms wahrnehmbar ist. Dadurch kann die Person die äußere Hüllkurve 13 mit Bereichen der intakten Netzhaut wahrnehmen, die das Skotom unmittelbar umgeben. Die äußere Hüllkurve 13 umrahmt also in der Wahrnehmung der Person den Bereich des Skotoms.

Das Anzeigen der äußeren Hüllkurve 13 als Umrahmung des Skotoms stellt ein präzises und intuitives Feedback bezüglich der Größe und Form des Skotoms für die Person bereit.

Weiterhin kann ein Training des bevorzugten retinalen Fixationsorts Pₚ der Person mittels der Anzeigeeinheit 1 durchgeführt werden, während die äußere Hüllkurve 13 für die Person wie oben beschrieben fortwährend auf der Anzeigeeinheit 1 als Umrahmung des Skotoms wahrnehmbar ist. Das Training des bevorzugten retinalen Fixationsorts Pₚ wird beispielsweise mit dem Ziel durchgeführt, die Position des bevorzugten retinalen Fixationsorts Pₚ zum ersten optimalen bevorzugten retinalen Fixationsort Pᵥ₁ für die spezifische Sehaufgabe v1 zu verlagern. Die äußere Hüllkurve 13 kann dabei zusätzlich zu einem Szenario einer virtuellen Realität oder einer erweiterten Realität auf der Anzeigeeinheit 1 angezeigt werden.

Es hat sich gezeigt, dass die Kombination eines Szenarios einer virtuellen Realität oder einer erweiterten Realität mit der Anzeige der äußeren Hüllkurve 13 als Umrahmung des Skotoms das Training des bevorzugten retinalen Fixationsorts Pₚ insbesondere bei einer Verwendung eines Head-Mounted Displays als Anzeigeeinheit 1 besonders effektiv macht.

### Bezugszeichenliste

- 1: Anzeigeeinheit
- 2: Fixierpunkt
- 3: Abtastpunkt
- 4: Polarachse
- 10: Gesichtsfeld karte
- 11: Polarsektor
- 12: äußere Grenzlinie
- 13: äußere Hüllkurve

- (ρ, θ): Polarkoordinaten
- P: subjektive Sichtbarkeit
- F: Fovea
- Eye(x,y): fortwährend erfassten Augenausrichtung
- Pₚ: bevorzugter retinaler Fixationsort
- v1: erste spezifische Sehaufgabe
- v2: zweite spezifische Sehaufgabe
- Pᵥ₁: erster optimaler bevorzugter retinaler Fixationsort
- Pᵥ₂: zweiter optimaler bevorzugter retinaler Fixationsort
- (ρ_{Pv1}, θ_{Pv1}): erste Translationsvariablen
- (ρ_{Pv2}, θ_{Pv2}): zweite Translationsvariablen

## Patentansprüche

1. Verfahren zum Erfassen eines Gesichtsfelds einer ein Skotom aufweisenden Person, umfassend die folgenden Schritte:
fortwährendes Erfassen der Augenausrichtung der Person mittels einer Erfassungseinheit,
punktweises Abtasten des Gesichtsfelds der Person, um sehtaugliche und sehuntaugliche Punkte im Gesichtsfeld der Person zu bestimmen,
Auffinden des Skotoms als einen Bereich mit einer Vielzahl von sehuntauglichen Punkten,
Berechnen einer äußeren Grenzlinie (12) des Skotoms,
Berechnen einer äußeren Hüllkurve (13), die die äußere Grenzlinie (12) des Skotoms in einem vorbestimmten Abstand umgibt,
Anzeigen der äußeren Hüllkurve (13) auf einer Anzeigeeinheit (1) unter Berücksichtigung der fortwährend erfassten Augenausrichtung derart, dass die äußere Hüllkurve (13) für die Person bei beliebiger Ausrichtung des Auges der Person auf die Anzeigeeinheit (1) als Umrahmung des Skotoms wahrnehmbar ist.

2. Verfahren nach Anspruch 1, umfassend den weiteren Schritt:
Bestimmen einer Position eines bevorzugten retinalen Fixationsortes (Pₚ).

3. Verfahren nach Anspruch 1 oder 2, wobei das punktweise Abtasten des Gesichtsfelds der Person erfolgt, während die Person einen dauerhaft auf der Anzeigeeinheit (1) angezeigten Fixierpunkt (2) mit einem bevorzugten retinalen Fixationsort (Pₚ) zu fixieren hat.

4. Verfahren nach Anspruch 3, wobei das punktweise Abtasten durch einen zeitweise auf der Anzeigeeinheit (1) angezeigten Abtastpunkt (3) erfolgt, und wobei beim punktweisen Abtasten durch den Abtastpunkt (3) sukzessive einzelne Punkte des Gesichtsfelds in Polarkoordinaten mit Abstand ρ und Winkel θ bezüglich des bevorzugten retinalen Fixationsortes (Pₚ) erfasst werden.

5. Verfahren nach Anspruch 3 oder 4, wobei für jeden Punkt (ρ, θ) die subjektive Sichtbarkeit (P) durch die Person von der Person abgefragt wird, und wobei zu n derart erfassten Punkten (ρ, θ) eine 3xn-Matrix mit Einträgen (ρ, θ, P) und/oder als graphische Darstellung der n erfassten Punkten (ρ, θ) eine Gesichtsfeldkarte (10) gebildet wird.

6. Verfahren nach einem der Ansprüche 3 bis 5, wobei als Maß für die Qualität der Fixierung des auf der Anzeigeeinheit (1) angezeigten Fixierpunkts (2) durch den bevorzugten retinalen Fixationsort (Pₚ) eine Fixationsstabilität aus der fortwährend erfassten Augenausrichtung bestimmt wird.

7. Verfahren nach einem der Ansprüche 3 bis 6, wobei eine Korrektur der Polarkoordinaten eines Punkts (ρ, θ) des Gesichtsfelds durch die fortwährend erfasste Augenausrichtung erfolgt, falls der Fixierpunkt (2) im Moment des Abtastens durch den Abtastpunkt (3) nicht mit dem bevorzugten retinalen Fixationsort (Pₚ) fixiert ist.

8. Verfahren nach einem der vorhergehenden Ansprüche, wobei zusätzlich ein Bild vom Augenhintergrund des Auges der Person aufgenommen wird, wobei auf der Grundlage des Bilds die Position der Fovea (F) bestimmt wird, und
wobei das Bild und/oder die Position der Fovea (F) gespeichert werden.

9. Verfahren nach einem der vorhergehenden Ansprüche, wobei ein für eine spezifische Sehaufgabe optimaler Bereich aus den sehtauglichen Punkten im Gesichtsfeld der Person als Ersatz für den bevorzugten retinalen Fixationsort (Pₚ) bestimmt wird.

10. Verfahren nach einem der vorhergehenden Ansprüche, wobei die fortwährend erfasste Augenausrichtung, die sehtauglichen Punkte im Gesichtsfeld der Person, die sehuntauglichen Punkte im Gesichtsfeld der Person, die äußere Grenzlinie (12) des Skotoms, die äußere Hüllkurve (13), die Position des bevorzugten retinalen Fixationsortes (Pₚ), die Position der Fovea (F) und/oder der für eine spezifische Sehaufgabe (v1, v2) optimale Bereich mit einem Zeitpunkt der jeweiligen Bestimmung für das Auge oder die Augen der Person als aktuelle Daten der Person gespeichert werden, und
wobei aus den aktuellen Daten der Person, aus früheren entsprechend bestimmten Daten der Person und/oder aus entsprechend bestimmten Daten anderer Personen Aussagen für die weitere Entwicklung des Skotoms der Person, für die Position des bevorzugten retinalen Fixationsortes (Pₚ) der Person und/oder für den für eine spezifische Sehaufgabe (v1, v2) optimalen Bereich der Person erzeugt werden.

11. Verfahren nach Anspruch 10, wobei die Aussagen für die weitere Entwicklung des Skotoms der Person, für die Position des bevorzugten retinalen Fixationsortes (Pₚ) der Person und/oder für den für eine spezifische Sehaufgabe (v1, v2) optimalen Bereich der Person aus den aktuellen Daten der Person, aus früheren entsprechend bestimmten Daten der Person und/oder aus entsprechend bestimmten Daten anderer Personen mittels künstlicher Intelligenz erzeugt werden.

12. Verfahren nach einem der vorhergehenden Ansprüche, wobei das Skotom ein Zentralskotom ist.

13. Vorrichtung zum Erfassen eines Gesichtsfelds einer ein Skotom aufweisenden Person, umfassend
eine Erfassungseinheit zum fortwährenden Erfassen der Augenausrichtung der Person,
eine Anzeigeeinheit (1) zum Anzeigen von visueller Information, wobei die Anzeigeeinheit (1) in einer definierten und/oder bestimmbaren räumlichen Beziehung zur Position des Auges der Person angeordnet ist, und
eine Steuereinheit zum Durchführen eines Verfahrens nach einem der vorhergehenden Ansprüche.

14. Vorrichtung nach Anspruch 13, wobei das Skotom ein Zentralskotom ist.

15. Vorrichtung nach Anspruch 13 oder 14,
wobei die Anzeigeeinheit (1) ein Bildschirm ist, und
wobei die Vorrichtung weiterhin Mittel zur Platzierung des Kopfs der Person in einer relativ zum Bildschirm definierten Position und/oder Mittel zur Bestimmung einer Position des Kopfs relativ zum Bildschirm umfasst.

16. Vorrichtung nach einem der Ansprüche 13 bis 15,
wobei die Anzeigeeinheit (1) ein sphärisch geformter Bildschirm ist.

17. Vorrichtung nach Anspruch 13 oder 14,
wobei die Anzeigeeinheit (1) und die Erfassungseinheit in einer am Kopf der Person befestigten Kopfeinheit vorgesehen sind.

18. Computerprogramm mit auf einem Prozessor ablauffähigem Programmcode zum Ausführen der Verfahrensschritte eines Verfahrens nach einem der Ansprüche 1 bis 12 auf einer Vorrichtung nach einem der Ansprüche 13 bis 17.

19. Nichttransitorisches Speichermedium mit einem darauf gespeicherten Computerprogramm nach Anspruch 18.

## Claims

1. Method for capturing a visual field of a person having a scotoma, said method comprising the following steps:
continuously capturing the eye alignment of the person by means of a capturing unit,
sampling the visual field of the person point by point in order to determine points suitable and not suitable for sight in the visual field of the person,
finding the scotoma as a region with a multiplicity of points not suitable for sight,
calculating an outer boundary line (12) of the scotoma, calculating an outer enveloping curve (13), which surrounds the outer boundary line (12) of the scotoma at a predetermined distance,
displaying the outer enveloping curve (13) on a display unit (1) with the continuously captured eye alignment being taken into account in such a way that the outer enveloping curve (13) is perceivable as a frame of the scotoma by the person in the case of any alignment of the eye of the person onto the display unit (1).

2. Method according to Claim 1, comprising the further step of:
determining a position of a preferred retinal locus for fixation (Pₚ).

3. Method according to Claim 1 or 2, wherein the point-by-point sampling of the visual field of the person is implemented while the person should fixate on a fixation point (2), permanently displayed on the display unit (1), with a preferred retinal locus for fixation (Pₚ).

4. Method according to Claim 3, wherein the point-by-point sampling is implemented by a sampling point (3) that is intermittently displayed on the display unit (1), and wherein successive individual points of the visual field are captured in polar coordinates with distance ρ and angle θ in respect of the preferred retinal locus for fixation (Pₚ) within the scope of the point-by-point sampling by the sampling point (3).

5. Method according to Claim 3 or 4, wherein the person is questioned in relation to the subjective visibility (P) of each point (ρ, θ) to the person, and wherein a 3xn matrix with entries (ρ, θ, P) is formed for n points (ρ, θ) captured in this way and/or a visual field map (10) is formed as a graphical representation of the n captured points (ρ, θ).

6. Method according to any one of Claims 3 to 5, wherein a fixation stability is determined from the continuously captured eye alignment as a measure for the quality of the fixation by the preferred retinal locus for fixation (Pₚ) on the fixation point (2) displayed on the display unit (1).

7. Method according to any one of Claims 3 to 6, wherein a correction of the polar coordinates of a point (ρ, θ) in the visual field is implemented by the continuously captured eye alignment should the fixation point (2) at the moment of sampling by the sampling point (3) not be fixated by the preferred retinal locus for fixation (Pₚ).

8. Method according to any one of the preceding claims, wherein an image of the fundus of the eye of the person is additionally recorded,
wherein the position of the fovea (F) is determined on the basis of the image, and
wherein the image and/or the position of the fovea (F) are stored.

9. Method according to any one of the preceding claims, wherein an optimal region for a specific visual task is determined from the points suitable for sight in the visual field of the person as a replacement for the preferred retinal locus for fixation (Pₚ).

10. Method according to any one of the preceding claims, wherein the continuously captured eye alignment, the points suitable for sight in the visual field of the person, the points unsuitable for sight in the visual field of the person, the outer boundary line (12) of the scotoma, the outer enveloping curve (13), the position of the preferred retinal locus for fixation (Pₚ), the position of the fovea (F) and/or the optimal region for a specific visual task (v1, v2) are stored with a time of the respective determination for the eye or the eyes of the person as current data of the person, and wherein statements in relation to the further development of the scotoma of the person, the position of the preferred retinal locus for fixation (Pₚ) of the person and/or the optimal region of the person for a specific visual task (v1, v2) are produced from the current data of the person, from earlier correspondingly determined data of the person and/or from correspondingly determined data of other persons.

11. Method according to Claim 10, wherein the statements in relation to the further development of the scotoma of the person, the position of the preferred retinal locus for fixation (Pₚ) of the person and/or the optimal region of the person for a specific visual task (v1, v2) are produced by means of artificial intelligence from the current data of the person, from earlier correspondingly determined data of the person and/or from correspondingly determined data of other persons.

12. Method according to any one of the preceding claims, wherein the scotoma is a central scotoma.

13. Apparatus for capturing a visual field of a person having a scotoma, said apparatus comprising a capturing unit for continuously capturing the eye alignment of the person,
a display unit (1) for displaying visual information, wherein the display unit (1) is arranged with respect to the position of the eye of the person in a defined and/or determinable spatial relationship, and
a control unit for carrying out a method according to any one of the preceding claims.

14. Apparatus according to Claim 13, wherein the scotoma is a central scotoma.

15. Apparatus according to Claim 13 or 14,
wherein the display unit (1) is a screen and
wherein the apparatus furthermore comprises means for placing the head of the person in a position that is defined relative to the screen and/or means for determining a position of the head relative to the screen.

16. Apparatus according to any one of Claims 13 to 15, wherein the display unit (1) is a spherically formed screen.

17. Apparatus according to Claim 13 or 14,
wherein the display unit (1) and the capturing unit are provided in a head unit that is fastened to the head of the person.

18. Computer program with program code that is executable on a processor for carrying out the method steps of a method according to any one of Claims 1 to 12 on an apparatus according to any one of Claims 13 to 17.

19. Non-transitory storage medium with a computer program according to Claim 18 stored therein.

## Revendications

1. Procédé pour détecter un champ de vision d'une personne présentant un scotome, comprenant les étapes suivantes :
détection continue de l'orientation des yeux de la personne au moyen d'une unité de détection,
échantillonnage ponctuel du champ de vision de la personne afin de déterminer les points aptes à la vision et inaptes à la vision dans le champ de vision de la personne,
recherche du scotome sous la forme d'une zone comprenant une pluralité de points inaptes à la vision,
calcul d'une ligne de délimitation (12) extérieure du scotome,
détermination d'une courbe d'enveloppe (13) extérieure qui entoure la ligne de délimitation (12) extérieure du scotome à une distance prédéterminée,
affichage de la courbe d'enveloppe (13) extérieure sur une unité d'affichage (1) en tenant compte de l'orientation des yeux détectée continuellement, de sorte que la courbe d'enveloppe (13) extérieure peut être perçue par la personne comme un encadrement du scotome avec une orientation quelconque de l'œil de la personne sur l'unité d'affichage (1).

2. Procédé selon la revendication 1, comprenant l'étape supplémentaire suivante :
détermination d'une position d'un lieu de fixation rétinienne préférentiel (Pₚ).

3. Procédé selon la revendication 1 ou 2, l'échantillonnage ponctuel du champ de vision de la personne étant effectué pendant que la personne doit fixer un point fixe (2) affiché durablement sur l'unité d'affichage (1) avec un lieu de fixation rétinienne préférentiel (Pₚ).

4. Procédé selon la revendication 3, l'échantillonnage ponctuel étant effectué par le biais d'un point d'échantillonnage (3) affiché temporairement sur l'unité d'affichage (1), et lors de l'échantillonnage ponctuel par le biais du point d'échantillonnage (3), des points individuels successifs du champ de vision étant détectés dans des coordonnées polaires avec un écart ρ et un angle θ par rapport au lieu de fixation rétinienne préférentiel (Pₚ).

5. Procédé selon la revendication 3 ou 4, la visibilité subjective (P) par la personne étant demandée à la personne pour chaque point (ρ, θ), et pour n points (ρ, θ) ainsi collectés, une matrice 3xn avec les éléments (ρ, θ, P) et/ou une carte de champ de vision (10) sous la forme d'une représentation graphique des n points (ρ, θ) collectés étant formées.

6. Procédé selon l'une des revendications 3 à 5, une stabilité de la fixation de l'orientation des yeux détectée continuellement étant déterminée en tant que mesure pour la qualité de la fixation du point fixe (2) affiché sur l'unité d'affichage (1) par le lieu de fixation rétinienne préférentiel (Pₚ).

7. Procédé selon l'une des revendications 3 à 6, une correction des coordonnées polaires d'un point (ρ, θ) du champ de vision étant effectuée par l'orientation des yeux détectée continuellement dans le cas où le point fixe (2) n'est pas fixé avec le lieu de fixation rétinienne préférentiel (Pₚ) au moment de l'échantillonnage par le biais du point d'échantillonnage (3) .

8. Procédé selon l'une des revendications précédentes, une image du fond de l'œil de la personne étant en outre enregistrée, la position de la fovéa (F) étant déterminée en se basant sur l'image et l'image et/ou la position de la fovéa (F) étant mémorisées.

9. Procédé selon l'une des revendications précédentes, une zone optimale pour une tâche de vision spécifique étant déterminée à partir des points aptes à la vision dans le champ de vision de la personne en tant que substitut du lieu de fixation rétinienne préférentiel (Pₚ).

10. Procédé selon l'une des revendications précédentes, l'orientation des yeux détectée continuellement, les points aptes à la vision dans le champ de vision de la personne, les points inaptes à la vision dans le champ de vision de la personne, la ligne de délimitation (12) extérieure du scotome, la courbe d'enveloppe (13) extérieure, la position du lieu de fixation rétinienne préférentiel (Pₚ), la position de la fovéa (F) et/ou la zone optimale pour une tâche de vision spécifique (v1, v2) étant mémorisés avec un instant de la détermination respective pour l'œil ou les yeux de la personne en tant que données actuelles de la personne, et
des assertions pour la poursuite du développement du scotome de la personne, pour la position du lieu de fixation rétinienne préférentiel (Pₚ) de la personne et/ou pour la zone optimale pour une tâche de vision spécifique (v1, v2) de la personne étant générées à partir des données actuelles de la personne, à partir de données correspondantes déterminées antérieurement de la personne et/ou à partir de données correspondantes déterminées antérieurement d'autres personnes.

11. Procédé selon la revendication 10, les assertions pour la poursuite du développement du scotome de la personne, pour la position du lieu de fixation rétinienne préférentiel (Pₚ) de la personne et/ou pour la zone optimale pour une tâche de vision spécifique (v1, v2) de la personne étant générées à partir des données actuelles de la personne, à partir de données correspondantes déterminées antérieurement de la personne et/ou à partir de données correspondantes déterminées antérieurement d'autres personnes au moyen de l'intelligence artificielle.

12. Procédé selon l'une des revendications précédentes, le scotome étant un scotome central.

13. Dispositif pour détecter un champ de vision d'une personne présentant un scotome, comprenant une unité de détection destinée à détecter en continu l'orientation des yeux de la personne,
une unité d'affichage (1) destinée à afficher des informations visuelles, l'unité d'affichage (1) étant disposée selon une relation spatiale définie et/ou pouvant être déterminée par rapport à la position de l'œil de la personne et
une unité de commande destinée à mettre en œuvre un procédé selon l'une des revendications précédentes.

14. Dispositif selon la revendication 13, le scotome étant un scotome central.

15. Dispositif selon la revendication 13 ou 14, l'unité d'affichage (1) étant un écran et le dispositif comportant en outre des moyens destinés à placer la tête de la personne dans une position définie par rapport à l'écran et/ou des moyens destinés à déterminer une position de la tête par rapport à l'écran.

16. Dispositif selon l'une des revendications 13 à 15, l'unité d'affichage (1) étant un écran de forme sphérique.

17. Dispositif selon la revendication 13 ou 14, l'unité d'affichage (1) et l'unité de détection se trouvant dans une unité de tête fixée à la tête de la personne.

18. Programme informatique comprenant un code de programme exécutable sur un processeur pour exécuter les étapes d'un procédé selon l'une des revendications 1 à 12 sur un dispositif selon l'une des revendications 13 à 17.

19. Support de mémorisation non temporaire sur lequel est mémorisé un programme informatique selon la revendication 18.
